Europäisches Patentamt

(19) European Patent Office

Office européen des brevets

(11) Publication number: **0 223 356**
**B1**

# (12) EUROPEAN PATENT SPECIFICATION

(45) Date of publication of the patent specification:
27.06.90

(51) Int. Cl.⁵: **A61B 3/14**

(21) Application number: **86307101.5**

(22) Date of filing: **16.09.86**

(54) **Two-dimensional eye fundus scanning apparatus.**

(30) Priority: **17.09.85 US 777406**
**19.06.86 US 876230**
**19.06.86 US 876231**

(43) Date of publication of application:
**27.05.87 Bulletin 87/22**

(45) Publication of the grant of the patent:
**27.06.90 Bulletin 90/26**

(84) Designated Contracting States:
**AT BE CH DE FR GB IT LI LU NL SE**

(56) References cited:
**US-A- 4 170 398**
**US-A- 4 213 678**

**APPLIED OPTICS, vol. 19, no. 17, 1st September 1980, pages 2991-2997, Optical Society of America, New York, US; R.H. WEBB et al.: "Flying spot TV ophthalmoscope"**
**IEEE PROCEEDINGS OF THE INTERNATIONAL JOINT CONFERENCE ON PATTERN RECOGNITION, Munich, 19th-22nd October 1982, part 1, page 1226, IEEE, New York, US; J. BILLE et al.: "Laser scanning ophthalmoscope with active focus control"**
**JOURNAL OF OPTICS, vol. 15, no. 6, November/December 1984, pages 425-430, Paris, FR; J.**

(73) Proprietor: **EYE RESEARCH INSTITUTE OF RETINA FOUNDATION, 20 Staniford Street, Boston Massachusetts 02114(US)**

(72) Inventor: **Webb, Robert H., Old Concord Road, Lincoln Massachusetts(US)**
Inventor: **Hughes, George W., 239 Clinton Road, Brookline Massachusetts(US)**

(74) Representative: **Hughes, Brian Patrick et al, Graham Watt & Co. Riverhead, Sevenoaks, Kent TN13 2BN(GB)**

(56) References cited: (continuation)
**COHEN et al.: "Ophtalmoscope a balayage optique" IEEE TRANSACTIONS ON BIOMEDICAL ENGINEERING, vol. BME-28, no. 7, July 1981, pages 488-492, IEEE, New York, US; R.H. WEBB et al.: "Scanning laser ophthalmoscope"**

## Description

### FIELD OF THE INVENTION

This invention relates in general to optical instruments and methods, and more particularly to an instrument for scanning a surface with an optical beam, detecting the light emitted from the surface, and generating a two-dimensional representation of an image of the structure.

### BACKGROUND OF THE INVENTION

In the art of optical instruments, it is known to scan a surface to be imaged with a small light source, collect the light reflected from the illuminated spot and direct it to a detector which provides an output signal varying in time in correlation with the scanning of the illuminated spot across the surface. The detector output can be stored in a permanent storage medium or provided directly to a scanning display device, such as a television raster or a cathode ray tube display. By synchronizing the scanning operation of the illuminating source with the scanning of the display signals, a two-dimensional image is produced.

One such instrument is a scanning ophthalmoscope which produces an image of the fundus of the eye. It has been found that the use of a laser light source provides improved imaging in an ophthalmoscope. A laser scanning ophthalmoscope is described in U.S. Patent No. 4,213,678. One problem associated with ophthalmoscopes of the type described in U.S. Patent 4,213,678 is that the light collected, at the time the laser is illuminating a particular area on the retina, includes not only light reflected directly from that area, but also light scattered from other surfaces and materials within the eye. This scattered light can cloud or fog the image, since it represents light contributions from other than the specific illuminated area. In an ideal system, each small illuminated area of the target object being examined produces a corresponding image area in the output display, with a brightness or intensity related only to light reflected directly from that target area. In some situations, on the other hand, the scattered light by itself, to the degree that it can be separated from the light directly reflected from the illuminated target area, is useful for diagnostic purposes.

In a device as described in the noted patent, the entrance pupil for the scanning laser beam has a small cross sectional area within the pupil of the eye, typically 0.5 mm in diameter, whereas the exit aperture for the reflected light is the overall pupil of the eye, which typically is nine mm in diameter. The detector is placed in a plane conjugate to this exit aperture. In the embodiment described in the patent, the scanning is effected by deflection galvanometers. The horizontal galvanometer is driven at 15.75 kHz in order to match the horizontal scan frequency of a conventional television sweep, which preferably is used to display the output image. The vertical galvanometer is driven at 60 Hz to produce 525 lines per frame of the output image, again corresponding to the generation of a conventional television raster.

In a scanning ophthalmoscope of this type, the resolution in the raster display of the retinal image directly corresponds to the cross sectional area of the laser spot as it scans the retina. The contrast of the ultimate image depends, at least in part, upon the proportion of light received by the detector which is directly reflected from the illuminated area. Thus, to the extent that scattered light indirectly reaches the detector at the same time as it receives the light directly reflected from the illuminated area, the image is fogged and the contrast is reduced. The term "reflected" is used herein in a broad sense to refer to all optical energy returned by the target structure, it hence includes returned optical energy that results from both specular and diffuse reflection.

One technique used in some optical instruments to improve contrast for images of this type may be termed double scanning. According to this technique, the optical system is arranged to provide that the light reflected from the illuminated target area is selected with a scanning-like action related to the scanning of the incident illumination in such a manner that, at a given instant, the reflected light received by the detector is only that which is reflected from the illuminated target area. In effect, as applied to an ophthalmoscope, the fundus conjugate plane thereby allowing discrimination, at the conjugate retinal plane, between the light directly reflected from the retinal locus and that scattered either anteriorly or posteriorly, i.e. within the retina. This approach, however, has been deemed to be unsuitable for an instrument like the laser ophthalmoscope of the type described, because in that instrument the exit aperture for the reflected light is so large that the returning reflected beam was deemed to require an unduly large scanning element. Since, at the driving frequencies associated with a television raster, a deflection galvanometer is limited by mass considerations to a very small surface, in the order of three millimeters, a reflection galvanometer large enough to encompass the returning image has been deemed not feasible.

Another deflection element which has been used for scanning optical instruments is a multifaceted rotating polygon, which would have to rotate at sufficiently high speeds to produce a horizontal scan matching the television frequencies. However, once again the size of the facet required to encompass the image received from the eye's exit aperture is prohibitively large in terms of fabricating a polygonal reflector to rotate at the required speeds.

The acousto-optical deflector is also not available in a form considered suitable for the reflected beam in such an instrument, due to aperture limitations.

It is an object of the present invention to provide an ophthalmological instrument for providing a two-dimensional representation of reflection characteristics of structure within an eye essentially in response only to light reflected from the eye structure in a selected manner. In one particular embodiment, the image is created in response essentially to directly reflected light; and in another embodiment in response to indirectly reflected light.

## SUMMARY OF THE INVENTION

It has been found, in one practice, that a double scanning optical instrument can be constructed utilizing a laser source and a multifaceted polygonal reflector for horizontal scan, with a reflection galvanometer or other scanning element for vertical scan, where the facet size in the direction of scan for the polygonal reflector is necessarily small and the reflected beam from the exit aperture of the system is substantially larger than that facet dimension. In the illustrated embodiment described below, the small facets of the polygonal reflector intercept less than 20% of the reflected light from the exit aperture. However, unexpectedly, under these circumstances the instrument attains a significant improvement in contrast over a single scan system, despite the significant loss of throughput.

It has thus been found that an optical instrument, of the type which responds to light energy responsive to a scanned incident beam, can be provided with double scanning with at least one scan element having such a small size that the exit beam overfills it. That is, this scan element is of such small size that it intercepts only a portion of the exit beam. In spite of the resultant loss of exit beam energy, the double-scanning instrument attains images having significant improvements over those of prior instruments. An instrument according to the invention attains this improved performance even when configured to have a large optical exit aperture, as is often desired.

The present invention is a scanning ophthalmoscope for providing a two-dimensional output representation of reflection characteristics of the posterior region of the eye, said ophthalmoscope comprising, a laser source for generating a laser beam of defined cross sectional area which is small compared to the region which is to be scanned and to the pupil of the eye, an optical system for directing said laser beam through the pupil of the eye onto said fundus area, said optical system including, a first scanning element comprising a rotatable element having spaced facets for changing the direction of an incident laser beam and driving means for rotating said element so a facet thereof scans said laser beam along a first coordinate, reflective means for directing said laser beam from said facet of said first scanning element through a portion of the eye pupil onto the region thereby scanning said laser beam across said region, means for collecting light from said region through an exit aperture in the eye pupil and directing it via said reflective means to a detector to develop a time-varying output signal correlated with the scanning frequency of the incident laser beam, beam separating means through which said laser beam and said collected light pass, and output means for receiving said output signal and providing a two dimensional output representation of the scanned region, said ophthalmoscope being characterised in that said reflective means is positioned to produce a conjugate image of the eye pupil at said facet, and light collected from said region is directed back onto said rotatable element such that the pupil conjugate image overfills said facet so that said facet redirects only a portion of the collected light to the detector for developing said time varying output signal.

## DESCRIPTION OF THE DRAWINGS

For a fuller understanding of the nature and objects of the invention, reference may be made to the following description and the accompanying drawings, in which:

FIGURE 1 is a diagrammatic representation of one embodiment of a scanning ophthalmoscope according to the invention;

FIGURES 2 and 3 are explanatory ray diagrams of optical beam features of the embodiment illustrated in FIGURE 1;

FIGURES 4 and 5 are explanatory ray diagrams of optical scan features of the embodiment of FIGURE 1;

FIGURE 6 is an explanatory ray diagram of the embodiment of FIGURE 1, where the optical system includes a diaphragm stop and the detector is repositioned;

FIGURE 6A is a view of the diaphragm stop of FIGURE 6;

FIGURE 7 is a diagrammatic representation of a modification to the embodiment of FIGURE 1;

FIGURE 8 is a block diagram of a portion of the ophthalmoscope of FIGURE 7;

FIGURE 9 is a diagrammatic representation of another embodiment of a scanning ophthalmoscope according to the invention;

FIGURES 10 and 10A are diagrammatic representations of a telescope magnified for insertion in any of the embodiments illustrated;

FIGURE 11 is a diagram of the relationship between the axis of rotation of the scanning element of the embodiment of FIGURE 1 and the allowable movement of the ophthalmoscope apparatus;

FIGURE 12 is a block diagram of an electronic circuit employed in the practice of the invention; and

FIGURE 13 is a block diagram of another electronic circuit employed in the practice of the invention.

## DESCRIPTION OF THE PREFERRED EMBODIMENT

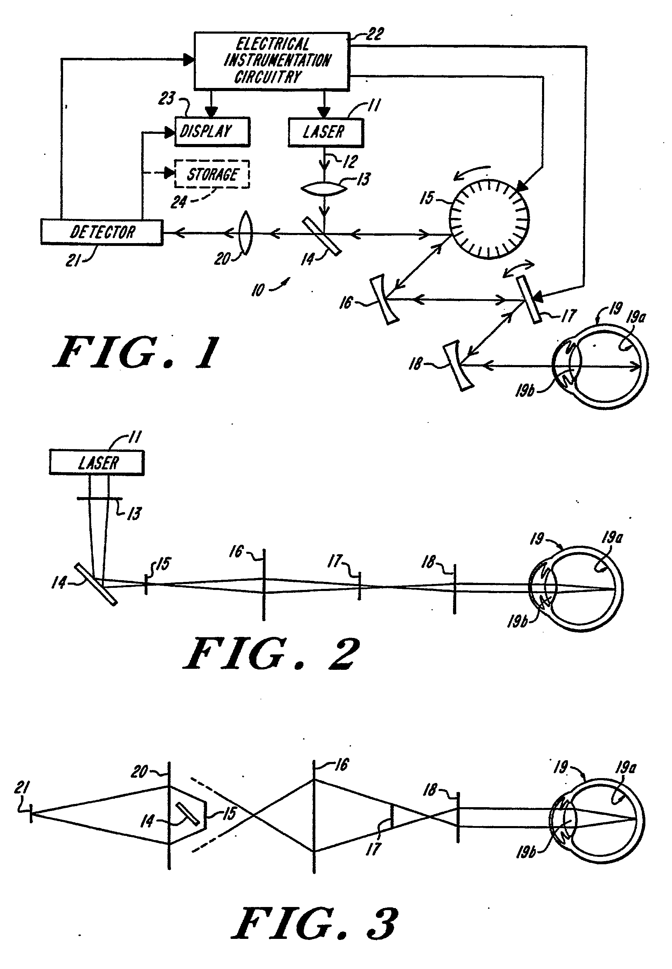

FIGURE 1 shows an embodiment of the invention in the form of an ophthalmoscope 10. A laser illumination source 11 produces a narrow incident light beam 12 which passes through a shaping lens system 13 which produces a slightly converging beam that impinges on a small turning mirror 14. The mirror 14 directs the incident laser beam onto facets of a multi-faceted rotating polygonal reflector scanner 15, which provides a horizontal scanning motion of the incident beam. The incident beam is reflected from this first stage scanning element onto a focusing mirror 16, which directs the beam onto the reflecting surface of a galvanometer reflector scanner 17 to produce a vertical scanning motion. From the galvanometer reflector scanner 17, which is a second stage scanning element, the laser input beam is directed onto a second focusing mirror 18, for focusing it onto the fundus 19 a of the eye 19 of a subject. The incident beam enters the eye at the crystalline lens 19 b.

The reflected light from the fundus 19 is directed back over a common portion of the foregoing optical input path, which includes focusing mirror 18, the second stage scanner 17, focusing mirror 16 and the first stage scanner 15. All of these common elements are mirrors and hence do not contribute reflections of the input beam back to the detector as noise background. The reflected output beam from the first stage scanner 15 in large part passes by the turning mirror 14 and hence separates from further traverse along the incident optical path. The output beam instead is directed through a focusing lens 20 and onto an optical detector 21.

The detector 21 is electrically connected to an electrical instrumentation unit 22 which provides electrical control signals to the laser source 11 and electrical drive signals to the scanning deflection elements 15 and 17. In essence, the instrumentation unit provides synchronization of the signals received at the scanning elements 15 and 17 so that the temporal order of the signals produced by the detector 21 can be correlated with the location of the scanned incident laser beam on the surface of the fundus. The control and synchronization which the instrumentation unit provides enables a two-dimensional display device 23, such as a television raster device, to form a two-dimensional display of an image of the eye fundus 19 a, in response to the electrical signal which the detector produces in response to the reflected optical energy it receives. The detector signal may be applied to a long term storage element 24, such as a video tape recorder, for subsequent readout and display. For a description of a suitable electrical timing and control circuit, reference is made to U.S. Patent 4,213,678.

## THE LASER GENERATOR

The laser 11 can be any suitable laser light source which provides emission at frequencies yielding appropriate contrast for the fundus, or other target. Typically, the laser 11 is an Argon-Krypton laser or Helium-Neon laser operated at a power level to produce an illumination irradiance of 100 microwatts per square centimeter at the fundus. The laser 11 may also be selected to emit in the infrared wavelength region to provide a scanning beam which does not require that the eye pupil be medically dilated to obtain an image of the fundus. For colour imaging two lasers of different wavelengths may be employed and converted into a single beam with a dichroic beam splitter.

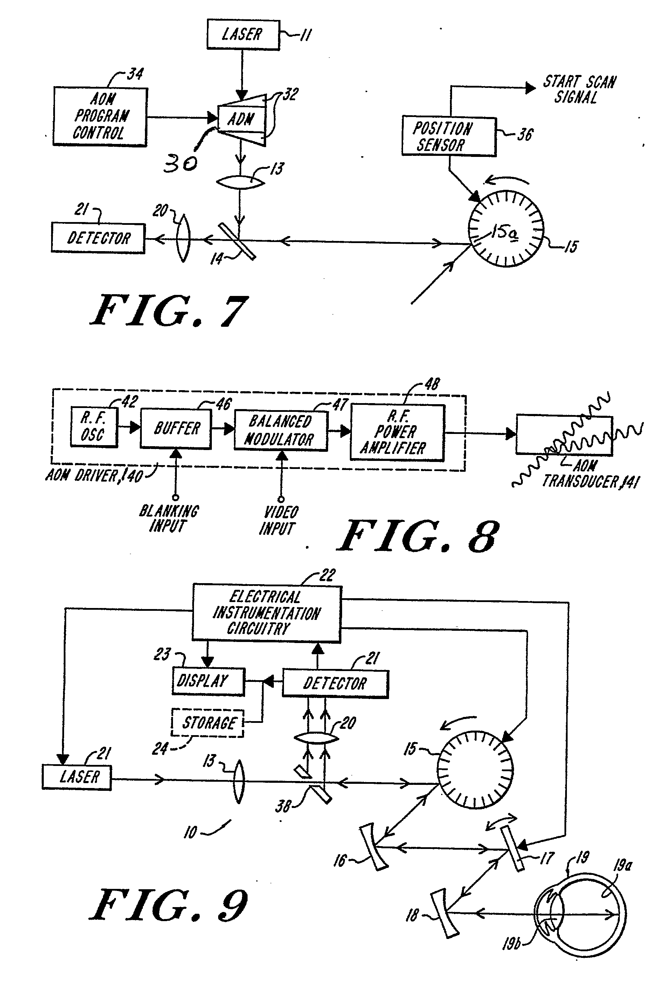

The laser beam, when it is emitting in the visible wavelength can also be arranged to present a graphic image, such as a cross in the scanning of the retina. FIGURE 7 illustrates a modification to the embodiment of FIGURE 1 in which additional elements are inserted between the laser 11 and the horizontal scanner 15. This embodiment includes an acousto-optic modulator (AOM) 30 for performing the graphic imaging function. The AOM receives a control input from a program control unit 34, which is typically a computer programmed to provide a signal timed to direct the laser beam emerging from the AOM away from the scanning path, thus blanking the scanning beam appropriately, to produce the image, a suitable computer being an IBM PC-XT made by International Business Machines, Yorktown Heights, New York with a Revolution 512 x 8 graphics peripheral card with gen lock, made by Number Nine Computer, Cambridge, Massachusetts. A program available for the graphic control is Media Cybernetics' Halo, by Media Cybernetics of Takoma Park, Md.

A visible graphic image may also be provided when an infrared laser is employed, by utilizing an incandescent light beam incident on the AOM. The incandescent source will have sufficient intensity to stimulate the patient's retina but will not affect the scanned output image.

Prisms 32 are placed in the beam between the laser and the AOM and after the AOM to allow lasers of different wavelengths to be used, while preserving the same Bragg angle relationship for the different wavelengths within the AOM to maintain the output beams from the AOM on the same optical axis.

FIGURE 8 is a block diagram of the acousto-optic modulator.

The acousto-optic modulator includes a driver unit 140 coupled to a transducer unit 141. The driver unit 140 includes an RF oscillator 42 typically operating at 40 MHz, followed by a buffer 46 which couples the oscillator to a balanced modulator 47. The output from the balance modulator 47 is coupled through an RF power amplifier 48 as the modulated RF output to the transducer element 140. The transducer element 141 is typically a glass crystal having Piezoelectric elements bonded to it to produce acoustic waves in the glass crystal. Optical waves incident on this crystal are then diffracted when the balanced modulator provides an output and remain undiffracted when there is no output from the balanced modulator, that is when the output from the driver 140 is blanked. The driver and transducer unit is commercially available from IntraAction Inc of Bellewood, Illinois, under the trade designation AEM40 & MOP402B. In the conventional acousto-optic modulator a video input is provided to the balanced modulator 47 thus controlling the output signal to effect the optical modulation; control signals are applied to this input from the graphics program control. However, a blanking input to the balanced modulator must be presented at times, not directly associated with the presentation of the graphics, in order completely to turn off the laser beam during the retrace of the display raster. This is done so that the laser beam does not impinge upon the patient's eye during this period, thus avoiding unnecessary irradiation of the patient's eye on distractingly visible retrace lines. This "absolute blanking" does not suffer from the requirement of careful adjustment typical of the balanced modulator circuit, but cannot achieve gray-scale modulation, since it is essentially on or off. In order to ensure that the inputs from the graphics control to the video input of the balanced modulator do not interfere with this retrace blanking, a retrace blanking signal is provided directly to buffer 46 to decouple the RF oscillator output 42 from the balanced modulator 47, thus disabling the driver unit 140 during this period.

The AOM diverts some of the beam energy into a first order (Bragg diffraction) beam at an angle typically about 15 mradians from the zero order beam. Either the original beam or the diffracted beam can be used to form the flying spot on the retina and its intensity is controllable over about three orders of magnitude by the AOM drive. The Bragg diffraction on which the modulation depends is from acoustic waves in a glass, of frequency 40 to 100 MHz. Two complications occur: because this is diffraction, it is inherently chromatic; and modulation of a high frequency carrier introduces other frequency components.

As above described the chromaticity is compensated with prisms 32 placed around the AOM. This brings both red and green beams to the glass at their preferred Bragg angles. The second prism is after the AOM to cause the beams to exit together, but the two prisms can be combined into one without serious problems. Minor adjustment at the combining dichroic beam splitter brings the two rasters into perfect alignment.

The trouble caused by the modulation itself is more subtle. When the RF carrier of, say, 40 MHz is turned off or on, lower frequencies are present for a few cycles. Lower frequencies deflect the beam at smaller angles, and a few cycles may well be a whole pixel. So, if the deflection is perpendicular to the fast (horizontal) scan direction, the beam moves off the raster line as it is turned off. In this orientation, all the line segments acquire little bends. This problem is solved if the AOM deflection angle is horizontal. The bends are still there, but they stretch the line segment out or tuck back into it, so that the only perception of them is that the leading edge of the segment is slightly softer and the trailing edge slightly sharper than expected.

## THE INPUT OPTICAL SYSTEM

The purpose of the input optical system is to scan the fundus with a narrow optical beam to sequentially illuminate small segmental areas across the fundus surface in a known pattern so that the reflected light detected in time sequence can be electrically converted to a two-dimensional representation of the reflection characteristics of the fundus. In one illustrative instrument, the input optical system forms the incident laser beam with a cross sectional area of substantially 0.5 mm diameter at the entrance pupil of the eye and focussed on the fundus to produce a spot approximately twelve microns in diameter. The horizontal scanning motion in the illustrated preferred embodiment is provided by a rotational scanner which is shown in the preferred embodiment as a multi-faceted polygonal reflector scanner 15 which is rotated by an electric motor at speeds sufficient to produce a scanning frequency of 15.75 kHz to be compatible with a TV sweep frequency. A polygon of (m) facets turns the incident laser beam through a scan angle of 720/m degrees. Thus, if, for example, there are twenty-four facets on the polygon, it must rotate at 40,000 rpm in order to generate the 15.75 kHz scanning frequency. In order to rotate at this speed the moment of inertia of the polygon must be kept small. In one practical embodiment, each facet is six mm wide. The polygonal rotating reflector of the scanner 15 can be obtained commercially from Lincoln Laser, Pheonix, Arizona, No. PO-24 (A Grade, G Grade). A holographic disk scanner, such as made by Holotech, Inc., which has spaced holographic facets may be substituted for this polygon reflector. The scan angle can be changed optically by any of the subsequent optical elements. One approach to modifying the field of view is to set the vertical manner to the same 28.8 degrees and then to modify the whole field of view at once. Resolution depends on the ratio of scan aperture to scan angle, so proper optical modification after the scan preserves the original resolution. With an input beam diameter of about 1 mm at the polygon, the available resolution is 794 spots, of which only 667 are used because of the 84% TV duty cycle. This is, in fact, about all that the available TV bandwidth can use. Once the resolution is fixed, at the polygon, the field of view can be increased or decreased by simple optical magnification. Increase of the field results in concurrent decrease of the beam diameter at the pupil, and this increases the spot size at the retina, so the resolution is unchanged.

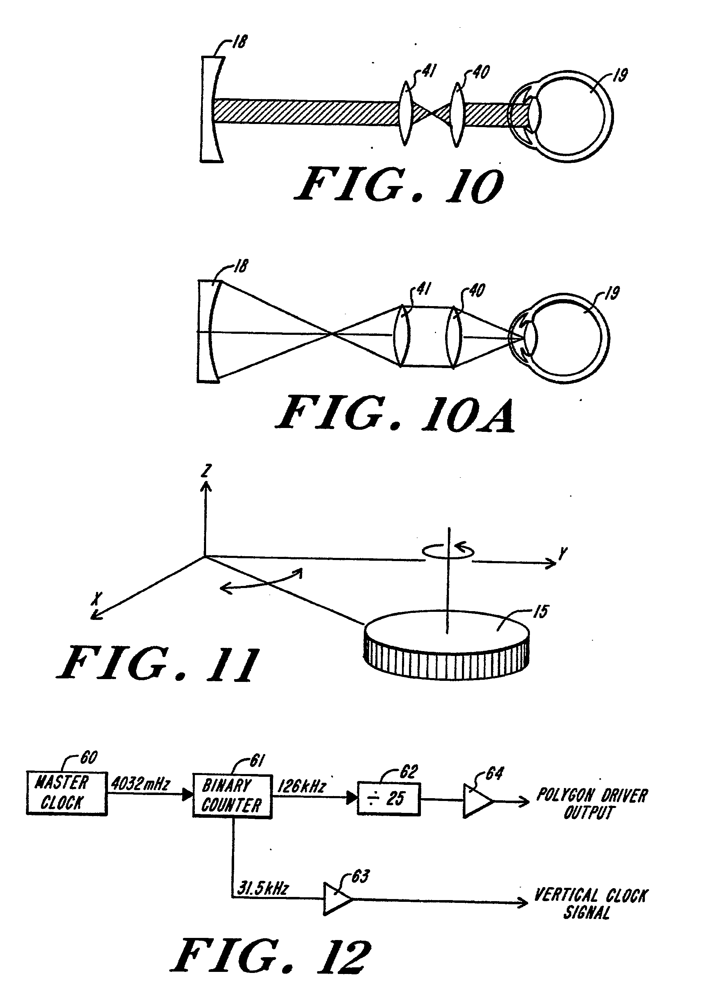

One method of changing the field size is to add an external telescope. This approach is illustrated in FIGURES 10 and 10A. FIGURE 10 is an illustration of the beam diagram of this system while FIGURE 10A illustrates the return reflection envelope. In FIGURE 10 lenses 40 and 41 are placed between the eye pupil and the mirror 18. Lens 40 is typically a 28-diopter ophthalmoscope lens and lens 41 may be a 14-diopter ophthalmoscope lens. By reversing the position of the lenses the field of view can be made smaller. If the distance between lenses 40 and 41 is adjusted to be unequal to the sum of the lenses' focal length, then refractive errors in the patient's eye can be compensated.

An advantage of this arrangement is that the telescope spacing adjusts beam focus, providing an independent compensation for patients' refractive error. This telescope system does, however, produce reflections. Four refractive surfaces intercept the incoming laser beam and reflect it back to the detector. Some of these reflections can be blocked by appropriately placed stops and some can be diminished or displaced by suitable choice of surface bend and tilt. The residuum can at least be localized to one small area of the picture. Since it is a moving picture, the clinician can easily look around such a single reflection. Finally, in the tightly confocal arrangement (small aperture at the retinal conjugate), the reflections substantially vanish, so that it is only in the afocal mode that they are a problem. Another method of changing the field size avoids these problems: element 18 can be placed in a position to increase or decrease the field at the retina. This is inconvenient to implement, but is a preferred embodiment if reflections are a problem.

The vertical scanning motion in the illustrated preferred embodiment is introduced by a deflection galvanometer 17 that provides a scan action which corresponds with the television vertical scan of 60 Hz. Galvanometer controls, such as those manufactured by General Scanning of Watertown, Massachusetts, are suitable for driving and controlling the position of the galvanometer mirror. The mirror 17 can, for example, be a type G120D General Scanning mirror.

With this structure and optical alignment in the instrument 10, the illustrated laser beam of 0.5 mm in diameter which it produces underfills each mirror facet of the polygon scanner 15, which, in the same illustrative embodiment, is six mm wide. The beam scanning pivots about a point in the plane of the eye's pupil.

The laser beam must be in focus at the retina, and the scan waist must be located (approximately) at the eyes of the pupil. Under these circumstances the spot size is appropriate for the available resolution, and the image will appear in focus at the TV screen even if it is not in focus at the confocal aperture. It is the focus of the incident beam which determines the picture's resolution and the focus of the return beam (at the confocal stop) which controls contrast. The fact that these controls are largely orthogonal is what allows flexibility as to mode of view.

The turning mirror 14 preferably is a stationary mirror reflector. It is small in size in order to produce a minimal shadow in the output beam, and hence preferably is only large enough to intercept the input beam which the focusing element 13 directs, via the turning mirror, to the first stage scanner 15. In the configuration shown the turning mirror acts as the beam separater between the input and reflected return beam.

In the embodiment illustrated in FIGURE 9 the laser beam is originally directed toward the polygon scanner 15. A mirror 38 with a central hole allows the laser beam to pass through it. The return reflected beam from the scanner 15 is then reflected by the annular portion of mirror 38 to the detector 21.

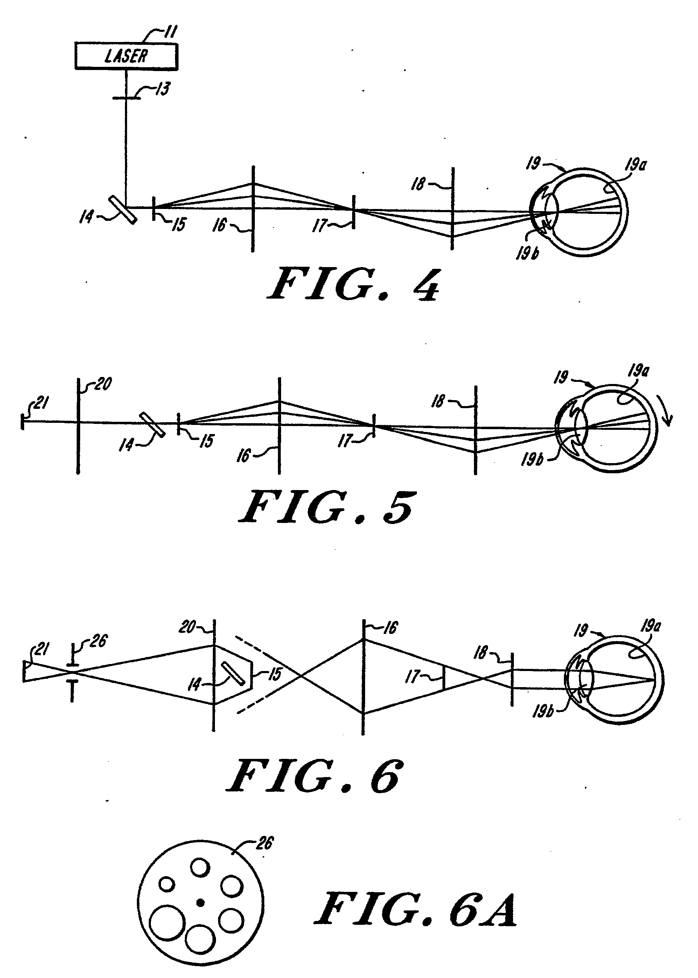

FIGURES 2 and 4 illustrate features of the input optical system. FIGURE 2 represents the input beam with the scanners assumed to be stationary in a neutral, non-deflecting, position. The narrow collimated incident beam 12 from the laser is, in this partial representation, shaped by the optical elements 13, 14, 16 and 18, aside from the eye 19 of the subject. The incident beam is in focus at the retina 19 a. The limiting aperture formed in this instance by the entrance pupil of the eye 19 is conjugate at the scanners 15 and 17.

FIGURE 4, which represents scan features of the input system, illustrates the input beam instantaneously as a single ray which each scanning element moves, as a function of time. The drawing shows, in effect, a time exposure. In the illustrated envelope, the beams intersect at the scanners and their conjugates, which, for the scanned input beam includes the entrance pupil. The scan angle is the full angle of this envelope in the plane of the scan.

The mirror 18 is large and spherical. Large, so that even at f/2 (for the scan) the eye's pupil is far back from the optics. With human subjects there are some inflexible dimensions. The mirror is spherical because no aspheric is correct for both beam and scan systems at all points. That constraint can be understood by noting that the beam on one side of this mirror may be always collimated, no matter where it hits the mirror. So the mirror must have everywhere the same local curvature - which implies a sphere. Since the mirror is used off-axis, the scan system is then astigmatic.

The scan system astigmatism can be corrected by adjusting the separation between the horizontal and vertical scanners along the system's optic axis. The small spherical mirror 16 is used as a relay between the two scanners, for more flexibility. This mirror only focuses a line scan, so it can be tilted in the orthogonal plane, contributing no astigmatism. Both mirrors contribute coma, of course, so tilt angles are kept small.

## THE OUTPUT OPTICAL SYSTEM

As noted, a major portion of the output optical system has a common optical path with the input system. This common path includes both of the scanning elements 15 and 17. In the illustrated instrument, it also includes the two focussing elements 16 and 18. However, in the output system, the light reflected from facets 15 a of the rotating polygon scanner 15 passes around the turning mirror 14 and is incident on the detector optical system, which includes lens 20 and detector 21.

FIGURE 3 represents the output beam without regard to the scanning elements 15 and 17, i.e. in the same manner as the representation in FIGURE 2. As illustrated, the reflected beam from the fundus has an exit aperture large compared to the cross section of the scanning beam, preferably substantially the entire pupil of the eye, with a diameter of as much as nine mm. The image of this aperture at its conjugate plane also is nine mm. Absent magnification, the reflected output beam from the illuminated area on the fundus likewise is approximately nine mm in diameter at any conjugate of the exit pupil, which is where the scan elements 15 and 17 are located.

In this configuration, the central region of the eye's pupil is used as an entrance pupil and the remaining annulus an exit pupil, thus conforming to Gulstrand's principle. This means that the scanners, optically conjugate to the pupil, need to be big enough to intercept that larger return beam. For the vertical scanner which moves as a 60 Hz sawtooth, a 10 - 15 mm mirror is suitable.

With the polygon, however, the available aperture (the facet) both rotates with respect to the beam and moves across it. The incident 1 mm beam and a 6 mm facet on the polygon combine to give just about the 84% duty cycle required for a TV raster. But the return beam may be as much as 15 mm in diameter, overfilling the facet even at the centre of its sweep. This does lose light, but the facet is filled with signal light over most of its duty cycle, and therefore a very uniform fraction of the light from the annular exit pupil is recovered.

The ophthalmoscope 10 can have a small entrance pupil, as described above, due to the large radiance of the incident beam. The output beam, however, has relatively low radiance, and hence the provision of this large output pupil is desired to collect a maximal amount of output light energy. The large exit aperture hence enhances the high efficiency of the instrument. It also facilitates viewing a large portion of the eye fundus.

FIGURE 3 also illustrates, with exaggerated scale, that the output beam passes around the turning mirror 14, which hence casts a small shadow generally of low significance.

It is desirable to separate the incident and return beams as close to the polygon facet as possible in order to place the incident beam in the centre of the return beam and thus stop direct reflection from the cornea (and spectacles if desired) from reaching the detector.

FIGURE 5 represents scan aspects of the output beam, in the same manner as the scanned input beam representation in FIGURE 4. The scanned output rays intersect, and the envelope of the scanned rays has minimal cross-section, at the pupillary plane of the eye 19 and at the scanning elements 15 and 17; this is the same as for the scanned input beam, FIGURE 4. The former is at the plane of the exit pupil and the latter are at planes conjugate to it.

As also illustrated in FIGURE 3, the relatively large cross-section of the output beam overfills each facet on the polygonal reflector scanner 15. With the six mm facet width of the illustrated embodiment, this overfill corresponds to a loss of throughput of approximately 80%. However, the reflected output light beam which the scanners 15 and 17 direct to the detector 21 is directly reflected substantially exclusively from the illuminated segmental area of the fundus. The detector 21 hence receives a minimal level of scatter or other unwanted light energy. These features enable the instrument to attain a resultant improvement of contrast at the detector which is unexpectedly high, and to yield a substantial improvement in contrast in the resultant image.

The placement in the instrument 10 of the detector 21 at the retinal conjugate plane, as apparent in FIGURE 3, is advantageous because it allows the detector to have a small aperture. Optical detectors of this type have numerous advantages over large-aperture detectors. In particular, an avalanche diode detector 21 is highly suitable for use as the detector in this system.

FIGURES 6 and 6A illustrate an alternative embodiment in which a diaphragm stop 26 is placed in the return beam path at the retinal conjugate plane and the detector 21 is moved to the pupillary conjugate plane. In FIGURE 6 the envelope of the return beam is diagrammed. For convenience the diaphragm stop 26 can be formed as a disk with varying size openings (as illustrated in FIGURE 6A) so that the size of the diaphragm stop 26 may be varied. Detectors are best placed at pupillary conjugates, since pupils tend to be about detector size (a few millimeters) while the retinal spot size is likely to be ten times smaller. The detector of choice is a semiconductor, typically a 1 mm avalanche diode with an integral amplifier such as RCA C30950E (RCA, Ste Anne de Bellvue, Quebec, Canada). When this detector is placed at the pupillary plane the retinal conjugate plane can be used for the placement of the aperture which limits the amount of retinal surface the detector receives light from. Since the retinal conjugate is a magnification (about ten times) of the retina a 1 mm aperture at the retinal plane restricts the retinal area seen to approximately 0.1 mm. On the other hand, if the aperture is made 10 mm, the retinal area seen is so much larger than the illuminating spot that the system is really afocal. A third option of interest is to use a 10 mm aperture with a central 1 mm stop, giving a "dark field" view of the retina, in which only light indirectly reflected is detected. With a rotatable aperture disk 26, as shown in FIGURE 6A the view can be varied from tightly confocal to afocal or dark field. The same disk can be arranged to carry filters for various wavelengths. Following the retinal plane a simple 10X microscope objective (not shown) can be used to bring the pupil back down to 1 mm for a match to the avalanche diode.

If the polygonal reflector 15 is formed with twenty-five facets, distortions due to facet-to-facet and other variations remain stationary in the displayed raster image, since it is evenly divisible into 525 television lines. For this reason, it is deemed preferable that the polygonal scanner have a number of reflective facets equal to an integral multiple of twenty-five. For different raster scan frequencies, a different number of facets would be appropriate. The controlling factor is that the number of reflecting facets should be integrally divisible into the number of raster lines. Further, as described above, there is a common optical path from the horizontal scanner 15 to the target object (in this example, the fundus of the eye) for the scanning beam and for the reflected light. Under these circumstances any reflection of the input laser beam from elements in the common optical path will appear as a noise signal to the detector. Accordingly, the focussing elements 16 and 18, as well as scanning elements 15 and 17, are front-surface mirrors.

While the instrument 10 has been described in terms of the advantages of de-scanning to produce signals corresponding only to light reflected directly from the illuminated target area, there are situations in which it is advantageous to look only at indirectly reflected light. This can be accomplished by moving the detector off the optical axis of the system so that it is in effect looking at target areas displaced from the direct illumination of the input beam. It has been found that information provided from these reflections also is useful in determining characteristics of an eye fundus. An alternative arrangement for attaining this reponse to only indirect illumination is to image on the detector a target area concentric with, and larger than, the illuminated area, and to mask light reflected from the illuminated area, e.g. with a dark-field or central stop.

Moreover, if the detector is moved axially, the plane of the image can be moved to positions anterior to the retinal surface and thus various types of floaters, such as vitreous spots and strands may become visible in the image. Similarly, movement of the image plane to posterior, sub-surface positions enables the instrument to image interior structure of the eye fundus.

The 15.75 kHz horizontal scan frequency and the 60 Hz vertical scan frequency described above for the illustrated embodiment are for use with television standards adopted for the USA. These values can be selected to suit other standards in practice in other countries. For example, the standard which operates at 625 lines per frame, requires the same 15.75 kHz horizontal scan frequency and a 50.4 Hz vertical scan frequency.

As a practical matter it is desirable to leave the patient comfortably stationary (in a head rest) and for the physician to move the ophthalmoscope to change the angle of the entrance beam. This means moving sources, detectors, optics and scanners. With a polygon rotating at 40,000 rpm, gyroscopic considerations must be addressed. To avoid gyroscopic torques the polygon must be moved only parallel to or perpendicular to its axis of spin. In the present embodiment a conventional fundus camera mount is used to support the ophthalmoscope and its motion can be controlled over short distances by a joy stick. Since the mount translates the polygon along X, Y or Z axes, and rotates it about the Z axis, as illustrated in FIGURE 11, none of the motions tilt the spinning axis Z of the polygon 15 and consequently there are no gyroscopic torques on the bearings.

In FIGURE 12 there is illustrated an oscillator clock supply which provides a polygon driver output signal and a vertical clock output signal. The oscillator includes a crystal controlled master clock 60, typically operated at 4.032 mHz. The output of the clock 60 is provided to a binary counter 61 and a 126 kHz signal from the binary counter is provided to a divide by 25 circuit 62, the output of which provides the polygon driver output signal. A second signal is taken from binary counter 61 at 31.5 kHz and this is the vertical clock output signal.

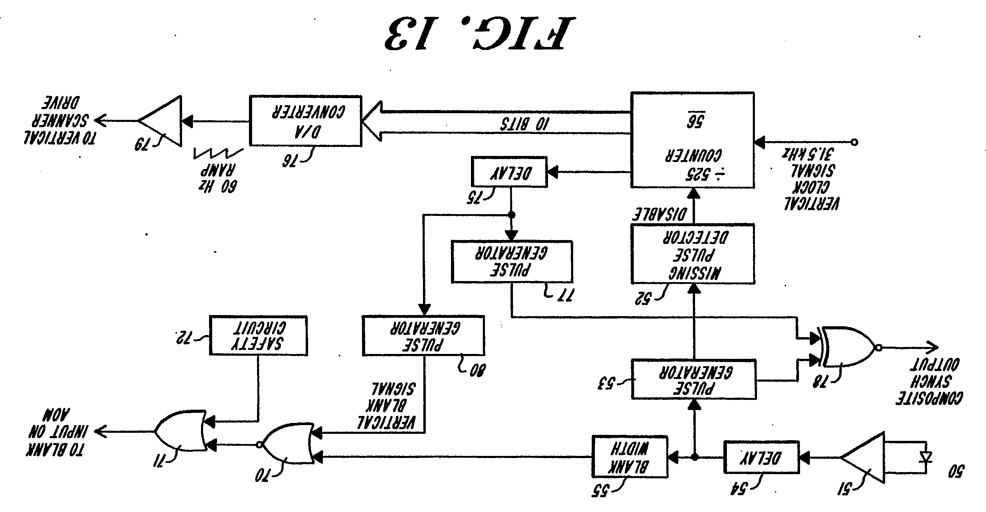

FIGURE 13 is a block diagram illustrating the manner in which the start of scan pulses generated from the pin diode 50 are processed to produce the blanking input for the AOM, the composite synch output for the monitor and other peripherals, and the vertical scanner drive. The output from the pin diode 50 is supplied through amplifier 51, delay circuit 54 and blank width control element 55 as one input to NOR gate 70. The delay unit 54 is arranged to equal the time required for the facet to rotate from the sensing position into the position where it intercepts the laser beam for scanning. Width circuit 55 provides for a pulse which is adjusted to be wide enough to cause blanking from the time one raster scan ends until the time the next raster scan is to begin. A second input to the NOR gate 70 is provided from the vertical synch signal. This signal is derived from counter 56 which is driven by the vertical clock signal. The vertical synch signal is processed through a delay unit 75 and a pulse generator 77. The output from NOR gate 70 is coupled through OR gate 71 to the blanking input on the AOM. A second input to the OR gate 71 is provided from a safety circuit 72. There are conventional safety circuits described in the literature, the purpose of which is to provide an output signal whenever a fault or failure in the overall ophthalmoscope is sensed. Thus, by coupling the safety circuit signal through the OR gate 71, it causes the laser beam to be diffracted away from the patient when such external faults occur.

The signal from delay 54 is provided to pulse generator 53, which has its output coupled to missing pulse detector 52. The latter circuit generates an output signal when there is no starter scan pulse. This output signal is provided as a disabling signal to counter 56.

Another output is taken from counter 56 to a digital to analog converter 76 which provides a 60 Hz ramp output signal to amplifier 79 to serve as a drive signal for the vertical scanning element. Thus the disabling signal to counter 56 ensures that the vertical scanner is not operating when there is no start scan pulse detected, that is, when the polygon is not rotating at proper speed.

The output from pulse generator 53, together with the output from pulse generator 77 is provided to EXCLUSIVE OR gate 78 to produce an output designated "comp synch output". The comp synch output is provided as a synchronizing signal to the television monitor and similar peripheral devices. It is also provided as an output to the computer controlling the presentation of the graphics to time that unit in relation to the start scan signals.

While the invention has been described in terms of an ophthalmoscope embodiment, the same principles can apply to the imaging of reflection characteristics of planes and structures other than the fundus of an eye with enhancement of the contrast characteristics of the representation. Note that the optical system of an instrument according to the invention does not focus the image of the object being scanned to produce an output image, but rather converts a selected portion of the reflected light to a time varying electrical signal, which can then be used to drive a synchronized imaging device and reproduce a representative visible image of the area being scanned.

## Claims

1. A scanning ophthalmoscope for providing a two dimensional output representation of reflection characteristics of the posterior region of the eye, said ophthalmoscope comprising,
a laser source (11) for generating a laser beam of defined cross sectional area which is small compared to the region (19a) which is to be scanned and to the pupil of the eye,
an optical system for directing said laser beam through the pupil of the eye onto said fundus area, said optical system including,

a first scanning element comprising a rotatable element (15) having spaced facets for changing the direction of an incident laser beam and driving means for rotating said element so a facet thereof scans said laser beam along a first coordinate,

reflective means (18) for directing said laser beam from said facet of said first scanning element through a portion of the eye pupil onto the region (19a) thereby scanning said laser beam across said region,

means for collecting light from said region through an exit aperture in the eye pupil and directing it via said reflective means (18) to a detector (21) to develop a time-varying output signal correlated with the scanning frequency of the incident laser beam,

beam separating means (14) via which said laser beam and said collected light pass, and

output means (23) for receiving said output signal and providing a two dimensional output representation of the scanned region,

said ophthalmoscope being characterised in that said reflective means is positioned to produce a conjugated image of the eye pupil at said facet, and light collected from said region is directed back onto said rotatable element such that the pupil conjugate image overfills said facet so that said facet redirects only a portion of the collected light to the detector (21) for developing said time varying output signal.

2. An ophthalmoscope as claimed in claim 1, characterised in that said first scanning element has a dimension along the coordinate of scan and a path of travel of each facet of said scanning element such that each facet sweeps across substantially the entire cross section of said reflected beam along the coordinate of scan while reflecting light back toward and incident upon said detector.

3. An ophthalmoscope as claimed in claim 1 or claim 2, characterised in that said reflective means is a focussing mirror (18).

4. An ophthalmoscope as claimed in any preceding claim, characterised in that said beam separating means (14) is positioned to direct the input laser beam toward said first scanning element, and to direct reflected light from said scanned region toward said detector (21), said beam separating means including a central stop for said reflected light, said central stop being substantially the same cross-sectional size as said input laser beam cross section, said detector means (21) being positioned optically beyond said beam separating means to receive said reflected light from said rotating element which passes back along the path toward said beam outside said stopped central position.

5. An ophthalmoscope as claimed in any preceding claim, characterised in that said first scanning element is a multifaceted polygon.

6. An ophthalmoscope as claimed in claim 3, characterised in that said focussing mirror (18) is tilted about one axis, the relative position ot said focussing mirror along the optical axis being adjusted to compensate for astigmatism produced by the tilt in the pupillary cross section of the envelope of the scanned beam.

7. An ophthalmoscope as claimed in claim 5, characterised in that the dimension of each polygon facet along the coordinate of scan, the dimension of the beam reflected from said fundus at the surface of said polygon facet, and the path of travel of each facet of said polygon along the coordinate of scan is such that a single facet sweeps across the entire cross sectional area of said beam in the direction of scan while reflecting light back toward said detector.

8. An ophthalmoscope as claimed in any preceding claim, characterised in that said beam separating means (14) is a turning mirror having a reflecting surface just large enough to encompass the defined cross sectional area of said laser beam, said turning mirror being positioned to intercept said laser beam and redirect it onto said first scanning element, said turning mirror being positioned to act as a central stop for said reflected light passing toward said detector.

9. An ophthalmoscope as claimed in any preceding claim, characterised in that a second scanning element (17) is arranged in optical alignment between said first scanning element and the eye to be scanned for moving said scanning laser beam in a direction normal to said first coordinate to effect a two-dimensional scan of said fundus area.

10. An opthalmoscope as claimed in claim 9 when dependent on claim 3, characterised by a second focussing mirror (16) positioned between said scanning elements (15 and 17) as a conjugate relay for directing said laser beam reflected from the facet of said first scanning element (15) onto the surface of said second focussing mirror, wherein one of said focussing mirrors is tilted about one axis, the relative positions of said focussing mirrors on the optical axis being adjusted to compensate for the astigmatism produced by said tilt in the pupillary cross section of the envelope of the scanned beam.

11. An ophthalmoscope as claimed in any preceding claim, characterised in that said laser beam has a wave length in the infrared region.

12. An ophthalmoscope as claimed in any preceding claim, characterised by
a diaphragm stop (26) positioned at the retinal conjugate plane in the reflected beam beyond a turning mirror,
said detector means (21) being positioned optically beyond said diaphragm stop at a pupillary image conjugate plane to receive said reflected light from said scanning element which passes back along the path toward said turning mirror, said turning mirror providing a stop for only the central portion of said reflected beam.

13. An ophthalmoscope as claimed in claim 12, characterised in that said diaphragm stop is constructed to have a variable aperture.

14. An ophthalmoscope as claimed in any preceding claim, characterised by an acousto-optical modulator (41) positioned to modulate said laser beam, and program means (34) for programming the modulation of said laser beam by the acousto-optical modulator to present specific graphic patterns in the scanning of said fundus area.

15. An ophthalmoscope as claimed in claim 14, characterised by prisms (32) optically coupled with said acousto-optic modulator (41) to provide for substantially the same angle of emission of laser beams of different incident wave length from the acousto-optic modulator at the same time preserving the Bragg relationship for the different wavelengths within the acousto-optic modulator.

16. An ophthalmoscope as claimed in claim 9, characterised in that said polygonal reflector (15) is rotated at a speed to produce a scanning frequency along said first coordinate on said fundus area of substantially 15.75 kHz and said second scanning element (17) produces a scanning motion in a direction normal to said first coordinate at substantially 50.4 or 60 Hertz and wherein said display means includes a television raster device.

17. An ophthalmoscope as claimed in claim 1, characterised in that said scanning laser beam is directed through a pivot point in a plane having a location selected relative to the laser beam for introducing the scanning laser beam into the eye being examined through a small portion only of the eye pupil, and wherein said scanning beam travels from said pivot point onto a wide-angle region of the fundus of the eye located with the eye pupil at said selected plane.

18. An ophthalmoscope as claimed in claim 15, characterised in that the size of the cross sectional area of said reflected beam is defined by the image at a conjugate plane of the exit aperture of said system, the exit aperture of said system being substantially larger than the entrance aperture for said scanning laser beam.

19. An ophthalmoscope as claimed in any preceding claim, characterised in that said detector is positioned to receive substantially only light reflected directly from the specific portion of the scanned area which is illuminated by the laser beam at any given time.

20. An ophthalmoscope as claimed in any of claims 1 to 18, characterised in that said detector is positioned to receive only light reflected from a specific portion of said scanned area which is illuminated indirectly from the portion of said scanned are which is illuminated directly at any given time.

21. An ophthalmoscope as claimed in claim 1, characterised in that said exit aperture is defined by the size of the pupil of said eye.

22. An ophthalmoscope as claimed in any preceding claim, characterised by means for sensing the position of successive facets of the first scanning element and providing a position signal indicating the time when each successive facet occupies the same position, said position signal being provided to the display means to control the timing of horizontal lines produced on said display means.

23. An ophthalmoscope as claimed in claim 22, characterised in that said display means is a two-dimensional television type raster.

24. An ophthalmoscope as claimed in any preceding claim, characterised by means for stopping said laser beam from scanning said eye fundus whenever a safety defect is sensed in said ophthalmoscope.

25. An ophthalmoscope as claimed in claim 22, characterised by means for stopping said laser beam from scanning said eye fundus, and wherein said position signal is provided to said means for stopping said laser beam to stop said laser beam from scanning during the time when said horizontal trace is not displayed.

26. An ophthalmoscope as claimed in any preceding claim, characterised in that said detector means is an avalanche diode.

27. An ophthalmoscope as claimed in claim 1, characterised by platform means for supporting said ophthalmoscope to provide movement of said ophthalmoscope for changing the angle at which said laser beam enters the pupil of the eye without changing the relative positions of the laser source, optical system and beam detector means, said platform means being mounted to allow translational movement of said ophthalmoscope only along two axes perpendicular to the axis of rotation of said rotating element and one axis parallel to said axis of rotation, and to allow rotational movement of said ophthalmoscope only about an axis parallel to the said axis of rotation.

28. An ophthalmoscope as claimed in claim 14, characterised by a control circuit,
said acousto-optical modulator having an electrical driver and an electromechanical transducer, said driver controlling the optical characteristics of said transducer to allow said laser beam to impinge upon said first scanning element or to be deflected away from said first scanning element,
wherein said driver comprises an RF oscillator, a balanced modulator having a video input, and a coupling circuit coupling said RF oscillator to said modulator,
said control circuit providing signals to said video input in accordance with a program from said program means.

29. An ophthalmoscope as claimed in claim 24, characterised in that said acousto-optical modulator comprises an electrical driver, and an electromechanical transducer, said electrical driver controlling the optical characteristics of said electromechanical transducer to allow said laser beam to impinge upon said first scanning element or to be deflected away from said first scanning element, and wherein said driver comprises an RF oscillator, a balanced modulator and a coupling circuit coupling said RF oscillator to said modulator, said laser beam being stopped from scanning by interrupting the coupling between said RF oscillator and said balanced modulator.

30. An ophthalmoscope as claimed in any of claims 1 to 10, characterised by a telescope having a magnification different than one positioned between said reflective means and said eye pupil for adjusting the beam focus on said fundus area.

31. An ophthalmoscope as claimed in any preceding claim, characterised in that said output means includes display means for displaying an image in response to said time varying signal and having variations in said image corresponding with variations in light directly reflected from the scanned object.

32. An ophthalmoscope as claimed in any preceding claim, characterised in that a second scanning element is positioned in the optical path of said input beam from said source and first scanning element for directing said input beam onto the object to be scanned and for moving said input beam in a direction normal to the direction of said first coordinate.

33. An ophthalmoscope as claimed in claim 32, characterised in that said second scanning element includes a reflecting galvanometer.

34. An ophthalmoscope as claimed in claim 5, characterised in that the rotation speed of said polygonal reflector is such that the scanning frequency of said input beam is substantially 15.75 kHz and wherein said output means includes a television-type raster imaging device.

35. An ophthalmoscope as claimed in claim 34, characterised by a second scanning element positioned in the optical path of said input beam from said source and first scanning element for directing said input beam onto the object to be scanned and for moving said input beam in a direction normal to the direction of said first coordinate, said second scanning element being operated to produce a scanning frequency in the direction normal to said first coordinate direction of substantially 50.4 or 60 Hz corresponding to a television frame rate.

36. An ophthalmoscope as claimed in claim 35, characterised in that said rotating polygonal reflector has a number of facets which is evenly divisible into 525.

37. An ophthalmoscope as claimed in claim 31, characterised in that said output means includes a multiple line raster imaging device and wherein said rotating polygonal reflector has a number of facets which is evenly divisible into the number of displayed raster lines.

38. A method of forming a two-dimensional output representation of reflection characteristics of a posterior region of the eye, said method comprising the steps generating a laser beam of defined cross sectional area which is small compared to the region which is to be scanned and to the pupil of the eye, directing said laser beam through the pupil of the eye onto said region by directing said beam at a facet of a rotatable scanning element to form a scanning laser beam along a first coordinate, and reflecting the scanning laser beam through a portion of the eye pupil onto said posterior region, collecting light from said region through the pupil and reflecting it back to a photodetector to develop a time-varying output signal correlated with the scanning frequency of the laser beam, and converting said output signal into a two-dimensional representation of the scanned region, wherein the method is characterised in that the step of collecting light from said region includes reflecting light from the region back to the rotatable scanning element which forms the scanning laser beam via a reflector which forms a conjugate image of the pupil at said facet, which image overfills said facet, so that the facet redirects only a portion of the collected light to the detector for developing said time-varying output signal.

39. A method as claimed in claim 38, characterised by the further step of compensating for scanning astigmatism.

40. A method as claimed in claim 38, characterised by the further steps of illuminating said object with said scanning input beam through a selected small input aperture, illuminating said detector with said output beam through a selected large exit aperture concentric with said input aperture, locating said facet of said first scanning device at a conjugate plane of said output aperture, and locating said detector at a conjugate plane of said output aperture.

41. A method as claimed in claim 38, characterised by the further step of locating means for detecting said output beam at a conjugate plane of the eye fundus.

**Patentansprüche**

1. Abtastophthalmoskop zur Lieferung einer zweidimensionalen Ausgangsdarstellung von Reflexionsmerkmalen des hinteren Bereichs des Auges, bestehend aus
einer Laserquelle (11) zur Erzeugung eines Laserstrahls mit einer begrenzten Querschnittsfläche, die im Vergleich zu dem Bereich (19a), der abzutasten ist, und zur Pupille des Auges klein ist, und
einem optischen System zum Hindurchleiten des Laserstrahls durch die Pupille des Auges auf den Fundusbereich, wobei das optische System besteht aus
einem ersten Abtastelement mit einem drehbaren Element (15), das mit Abstand voneinander angeordnete Facetten zum Ändern der Richtung eines einfallenden Laserstrahls besitzt, und mit Steuermitteln zum Drehen dieses Elements, so daß eine seiner Facetten den Laserstrahl auf einer ersten Koordinate abtastet,
einer Reflexionseinrichtung (18) zum Leiten des Laserstrahls von der Facette des ersten Abtastelements durch einen Teil der Augenpupille auf den Bereich (19a), um dadurch den Laserstrahl über diesen Bereich abzutasten,
einer Einrichtung zum Sammeln von Licht von diesem Bereich durch eine Austrittsöffnung in der Augenpupille und zu dessen Zuleitung über die Reflexionseinrichtung (18) zu einem Detektor (21), um ein mit der Abtastfrequenz des einfallenden Laserstrahls abgestimmtes zeitvariierendes Ausgangssignal zu entwickeln,
einer Strahlteilereinrichtung (14), die der Laserstrahl und das gesammelte Licht passieren, und
einer Ausgabeeinrichtung (23) zum Empfang des Ausgangssignals und zur Abgabe einer zweidimensionalen Ausgangsdarstellung des abgetasteten Bereichs,
und wobei das Ophthalmoskop dadurch gekennzeichnet ist, daß die Reflexionseinrichtung so positioniert ist, daß ein konjugiertes Bild der Augenpupille an der Facette produziert und von dem Bereich gesammeltes Licht zurück auf das rotierende Element geleitet wird, derart, daß das konjugierte Pupillenbild die Facette überfüllt, so daß die Facette nur einen Teil des gesammelten Lichts an den Detektor (21) zur Entwicklung des zeitvariierenden Ausgangssignals zurückleitet.

2. Ophthalmoskop nach Anspruch 1, dadurch gekennzeichnet, daß das erste Abtastelement eine solche Abmessung entlang der Abtastkoordinate und einen solchen Laufweg jeder Facette des Abtastelements besitzt, daß jede Facette über im wesentlichen den gesamten Querschnitt des reflektierten Strahls entlang der Abtastkoordinate fährt, während Licht zurück zum Detektor in diesen einfallend reflektiert wird.

3. Ophthalmoskop nach Anspruch 1 oder 2, dadurch gekennzeichnet, daß die Reflexionseinrichtung ein Fokussierspiegel (18) ist.

4. Ophthalmoskop nach einem beliebigen vorhergehenden Anspruch, dadurch gekennzeichnet, daß die Strahlteilereinrichtung (14) so positioniert ist, daß der Eintrittslaserstrahl zum ersten Abtastelement und reflektiertes Licht vom abgetasteten Bereich zu dem Detektor (21) geleitet wird, die Strahlteilereinrichtung einen zentralen Stop für das reflektierte Licht aufweist, der zentrale Stop im wesentlichen die gleiche Querschnittsgröße wie der Querschnitt des Eintrittslaserstrahls aufweist und die Detektoreinrichtung (21) optisch jenseits der Strahlteilereinrichtung zum Empfang des reflektierten Lichts von dem rotierenden Element angeordnet ist, das entlang dem Weg zu dem Strahl außerhalb der zentralen Stopstellung zurückläuft.

5. Ophthalmoskop nach einem beliebigen vorhergehenden Anspruch, dadurch gekennzeichnet, daß das erste Abtastelement ein Mehrfacetten-Polygon ist.

6. Ophthalmoskop nach Anspruch 3, dadurch gekennzeichnet, daß der Fokussierspiegel (18) um eine Achse geneigt und die Relativstellung des Fokussierspiegels entlang der optischen Achse für einen Ausgleich von Astigmatismus eingestellt ist, der durch die Neigung im Pupillenquerschnitt der Einhüllenden des Abtaststrahls erzeugt wird.

7. Ophthalmoskop nach Anspruch 5, dadurch gekennzeichnet, daß die Abmessung jeder Polygonfacette entlang der Abtastkoordinate, die Abmessung des vom Fundus an der Oberfläche der Polygonfacette reflektierten Strahls und der Laufweg jeder Facette des Polygons entlang der Abtastkoordinate derart bemessen ist, daß eine einzelne Facette über den gesamten Querschnittsbereich des Strahls in der Abtastrichtung läuft, während Licht zurück zum Detektor reflektiert wird.

8. Ophthalmoskop nach einem beliebigen vorhergehenden Anspruch, dadurch gekennzeichnet, daß die Strahlteilereinrichtung (14) ein Drehspiegel mit einer Reflexionsfläche ist, die gerade groß genug ist, um die begrenzte Querschnittsfläche des Laserstrahls zu umfassen, der Drehspiegel so angeordnet ist, daß er den Laserstrahl unterbricht und ihn zurück auf das erste Abtastelement leitet und daß der Drehspiegel so positioniert ist, daß er als ein zentraler Stop für das zum Detektor laufende reflektierte Licht wirkt.

9. Ophthalmoskop nach einem beliebigen vorhergehenden Anspruch, dadurch gekennzeichnet, daß ein zweites Abtastelement (17) in optischer Ausrichtung zwischen dem ersten Abtastelement und dem abzutastenden Auge für eine Bewegung des Abtastlaserstrahls in eine zur ersten Koordinate senkrechten Richtung zur Herbeiführung einer zweidimensionalen Abtastung des Fundusbereichs angeordnet ist.

10. Ophthalmoskop nach Anspruch 9 in Verbindung mit Anspruch 3, gekennzeichnet durch einen zweiten Fokussierspiegel (16) zwischen den Abtastelementen (15 und 17) als ein konjugiertes Relais zum Richten des von der Facette des ersten Abtastelements (15) reflektierten Laserstrahls auf die Oberfläche des zweiten Fokussierspiegels, wobei einer der Fokussierspiegel um eine Achse geneigt ist und die Relativstellungen der Fokussierspiegel auf der optischen Achse für einen Ausgleich von Astigmatismus eingestellt sind, der durch die Neigung im Pupillenquerschnitt der Einhüllenden des Abtaststrahls erzeugt wird.

11. Ophthalmoskop nach einem beliebigen vorhergehenden Anspruch, dadurch gekennzeichnet, daß der Laserstrahl eine Wellenlänge im Infrarotbereich hat.

12. Ophthalmoskop nach einem beliebigen vorhergehenden Anspruch, gekennzeichnet durch einen Blendenstop (26) an der konjugierten Netzhautebene im reflektierten Strahl jenseits eines Drehspiegels, wobei die Detektoreinrichtung (21) optisch jenseits des Blendenstops an einer konjugierten Pupillenbildebene angeordnet ist, um das reflektierte Licht von dem Abtastelement, das entlang dem Weg zum Drehspiegel zurückläuft, zu empfangen, und der Drehspiegel einen Stop nur für den zentralen Teil des reflektierten Strahls bildet.

13. Ophthalmoskop nach Anspruch 12, dadurch gekennzeichnet, daß der Blendenstop eine Ausbildung mit einer variablen Öffnung besitzt.

14. Ophthalmoskop nach einem beliebigen vorhergehenden Anspruch, gekennzeichnet durch einen akusto-optischen Modulator (41) zum Modulieren des Laserstrahls und eine Programmeinrichtung (34) zum Programmieren der Modulation des Laserstrahls durch den akusto-optischen Modulator zur Darbietung spezifischer graphischer Muster bei der Abtastung des Fundusbereichs.

15. Ophthalmoskop nach Anspruch 14, gekennzeichnet durch optisch mit dem akusto-optischen Modulator (41) gekoppelte Prismen (32) zur Herbeiführung im wesentlichen des gleichen Emissionswinkels von Laserstrahlen von unterschiedlicher Einfallwellenlänge vom akusto-optischen Modulator unter gleichzeitiger Wahrung des Bragg'schen Verhältnisses für die verschiedenen Wellenlängen im akusto-optischen Modulator.

16. Ophthalmoskop nach Anspruch 9, dadurch gekennzeichnet, daß der polygonale Reflektor (15) mit einer solchen Geschwindigkeit gedreht wird, daß eine Abtastfrequenz entlang der ersten Koordinate auf dem Fundusbereich von im wesentlichen 15,75 kHz erzeugt wird, und das zweite Abtastelement (17) eine Abtastbewegung in einer zur ersten Koordinate senkrechten Richtung mit im wesentlichen 50,4 oder 60 Hertz erzeugt, wobei die Anzeigeeinrichtung ein Fernsehrastergerät aufweist.

17. Ophthalmoskop nach Anspruch 1, dadurch gekennzeichnet, daß der Abtastlaserstrahl durch einen Drehpunkt in einer Ebene mit einer in bezug auf den Laserstrahl für eine Einführung des Abtastlaserstrahls in das untersuchte Auge durch nur einen kleinen Teil der Augenpupille gewählten Stellung hindurchgeleitet wird und der Abtaststrahl von dem Drehpunkt auf einen Weitwinkelbereich des Fundus des Auges läuft, der in der Augenpupille an der gewählten Ebene angeordnet ist.

18. Ophthalmoskop nach Anspruch 15, dadurch gekennzeichnet, daß die Größe der Querschnittsfläche des reflektierten Strahls durch das Bild an der konjugierten Ebene der Austrittsöffnung des Systems begrenzt ist und die Austrittsöffnung des Systems wesentlich größer als die Eintrittsöffnung für den Abtastlaserstrahl ist.

19. Ophthalmoskop nach einem beliebigen vorhergehenden Anspruch, dadurch gekennzeichnet, daß der Detektor so angeordnet ist, daß er im wesentlichen nur Licht empfängt, das direkt von dem speziellen Teil des abgetasteten Bereichs reflektiert wird, der von dem Laserstrahl zu einer gegebenen Zeit beleuchtet wird.

20. Ophthalmoskop nach einem der Ansprüche 1 bis 18, dadurch gekennzeichnet, daß der Detektor so angeordnet ist, daß er nur Licht empfängt, das von einem speziellen Teil des abgetasteten Bereichs reflektiert wird, der indirekt von dem Teil des abgetasteten Bereichs beleuchtet wird, der zu einer gegebenen Zeit direkt beleuchtet wird.

21. Ophthalmoskop nach Anspruch 1, dadurch gekennzeichnet, daß die Austrittsöffnung durch die Größe der Pupille des Auges gebildet ist.

22. Ophthalmoskop nach einem beliebigen vorhergehenden Anspruch, gekennzeichnet durch eine Einrichtung zum Abtasten der Position aufeinanderfolgender Facetten des ersten Abtastelements und zur Abgabe eines Positionssignals als Angabe der Zeit, zu der jede aufeinanderfolgende Facette die gleiche Position einnimmt, wobei das Positionssignal an die Anzeigeeinrichtung zur Kontrolle der Zeitsteuerung der auf der Anzeigeeinrichtung produzierten horizontalen Zeilen geliefert wird.

23. Ophthalmoskop nach Anspruch 22, dadurch gekennzeichnet, daß die Anzeigeeinrichtung ein zweidimensionales Fernsehtypraster ist.

24. Ophthalmoskop nach einem beliebigen vorhergehenden Anspruch, gekennzeichnet durch eine Einrichtung zum Stoppen des Laserstrahls im Abtasten des Augenfundus jedesmal dann, wenn ein Sicherheitsfehler im Ophthalmoskop festgestellt wird.

25. Ophthalmoskop nach Anspruch 22, gekennzeichnet durch eine Einrichtung zum Stoppen des Laserstrahls im Abtasten des Augenfundus, wobei das Positionssignal der Einrichtung zum Stoppen des Laserstrahls zugeführt wird, um den Laserstrahl in der Abtastung während der Zeit zu stoppen, in der die horizontale Bildspur nicht angezeigt wird.

26. Ophthalmoskop nach einem beliebigen vorhergehenden Anspruch, dadurch gekennzeichnet, daß die Detektoreinrichtung eine Zener-Diode ist.

27. Ophthalmoskop nach Anspruch 1, gekennzeichnet durch eine Plattform zur Abstützung des Ophthalmoskops zur Herbeiführung einer Bewegung des Ophthalmoskops zur Änderung des Winkels, mit dem der Laserstrahl in die Pupille des Auges eintritt, ohne Veränderung der Relativstellungen der Laserquelle, des optischen Systems und der Strahldetektoreinrichtung, wobei die Plattform so angebracht ist, daß sie eine Verschiebebewegung des Ophthalmoskops auf zwei zur Drehachse des Drehelements senkrechten Achsen und einer zur Drehachse parallelen Achse sowie eine Rotationsbewegung des Ophthalmoskops nur um eine zur Drehachse parallele Achse ermöglicht.

28. Ophthalmoskop nach Anspruch 14, gekennzeichnet durch eine Steuerschaltung, wobei der akusto-optische Modulator einen elektrischen Treiber und einen elektromechanischen Meßwandler aufweist, der Treiber die optischen Merkmale des Meßwandlers in der Weise steuert, daß es dem Laserstrahl möglich ist, das erste Abtastelement zu beaufschlagen oder vom ersten Abtastelement hinweg abgelenkt zu werden, der Treiber einen Hochfrequenzoszillator, einen abgeglichenen Modulator mit einem Videoeingang und eine den Hochfrequenzoszillator mit dem Modulator koppelnde Koppelschaltung umfaßt und die Steuerschaltung Signale an den Videoeingang gemäß einem Programm von der Programmeinrichtung liefert.

29. Ophthalmoskop nach Anspruch 24, dadurch gekennzeichnet, daß der akusto-optische Modulator einen elektrischen Treiber und einen elektromechanischen Meßwandler umfaßt, der elektrische Treiber die optischen Merkmale des elektromechanischen Meßwandlers so steuert, daß es dem Laserstrahl ermöglicht ist, das erste Abtastelement zu beaufschlagen oder vom ersten Abtastelement hinweg abgelenkt zu werden, der Treiber einen Hochfrequenzoszillator, einen abgeglichenen Modulator und eine den Hochfrequenzoszillator mit dem Modulator koppelnde Koppelschaltung umfaßt und der Laserstrahl in einem Abtasten durch Unterbrechung der Kopplung zwischen dem Hochfrequenzoszillator und dem abgeglichenen Modulator gestoppt wird.

30. Ophthalmoskop nach einem der Ansprüche 1 bis 10, gekennzeichnet durch ein Teleskop, das eine Vergrößerung unterschiedlich als bei einem zwischen der Reflexionseinrichtung und der Augenpupille angeordneten zur Einstellung des Strahlenfokus auf dem Fundusbereich aufweist.

31. Ophthalmoskop nach einem beliebigen vorhergehenden Anspruch, dadurch gekennzeichnet, daß die Ausgangseinrichtung eine Anzeigeeinrichtung zum Anzeigen eines Bildes infolge des zeitvariierenden Signals und Variationen in dem Bild entsprechend Variationen in direkt von dem abgetasteten Objekt reflektiertem Licht aufweist.

32. Ophthalmoskop nach einem beliebigen vorhergehenden Anspruch, dadurch gekennzeichnet, daß ein zweites Abtastelement im optischen Weg des Eintrittsstrahls von der Quelle und dem ersten Abtastelement angeordnet ist, um den Eintrittsstrahl auf das abzutastende Objekt zu richten und um den Eintrittsstrahl in eine Richtung senkrecht zur Richtung der ersten Koordinate zu bewegen.

33. Ophthalmoskop nach Anspruch 32, dadurch gekennzeichnet, daß das zweite Abtastelement einen Reflexionsgalvanometer umfaßt.

34. Ophthalmoskop nach Anspruch 5, dadurch gekennzeichnet, daß die Drehgeschwindigkeit des polygonalen Reflektors derart bemessen ist, daß die Abtastfrequenz des Eintrittsstrahls im wesentlichen 15,75 kHz beträgt, wobei die Ausgangseinrichtung ein Bildgerät mit Fernsehtypraster umfaßt.

35. Ophthalmoskop nach Anspruch 34, gekennzeichnet durch ein zweites Abtastelement im optischen Weg des Eintrittsstrahls von der Quelle und dem ersten Abtastelement zum Richten des Eintrittsstrahls auf das abzutastende Objekt und zur Bewegung des Eintrittsstrahls in einer zur Richtung der ersten Koordinate senkrechten Richtung, wobei das zweite Abtastelement derart betrieben wird, daß es eine Abtastfrequenz in der zur ersten Koordinatenrichtung senkrechten Richtung von im wesentlichen 50,4 oder 60 Hz entsprechend dem Fernsehbildraster erzeugt.

36. Ophthalmoskop nach Anspruch 35, dadurch gekennzeichnet, daß der rotierende Polygonreflektor eine Anzahl von Facetten aufweist, die sich gleichmäßig in 525 aufteilt.

37. Ophthalmoskop nach Anspruch 31, dadurch gekennzeichnet, daß die Ausgangseinrichtung ein Bildgerät mit Mehrfachzeilenraster aufweist und der rotierende Polygonreflektor eine Anzahl von Facetten besitzt, die gleichmäßig in die Anzahl angezeigter Rasterlinien aufteilbar ist.

38. Verfahren zum Bilden einer zweidimensionalen Ausgangsdarstellung von Reflexionsmerkmalen eines hinteren Bereichs des Auges, wobei das Verfahren die Schritte umfaßt, daß ein Laserstrahl von begrenzter Querschnittsfläche erzeugt wird, die im Vergleich zu dem Bereich, der abzutasten ist, und zur Pupille des Auges klein ist, der Laserstrahl durch die Pupille des Auges auf den Bereich gerichtet wird, indem der Strahl an eine Facette eines drehbaren Abtastelements zur Bildung eines Abtastlaserstrahls auf einer ersten Koordinate geleitet und der Abtastlaserstrahl durch einen Teil der Augenpupille auf den hinteren Bereich reflektiert wird, Licht von dem Bereich durch die Pupille gesammelt und dieses zurück zu einem Fotodetektor zur Entwicklung eines zeitvariierenden Ausgangssignals in Übereinstimmung mit der Abtastfrequenz des Laserstrahls reflektiert wird und das Ausgangssignal in eine zweidimensionale Darstellung des abgetasteten Bereichs umgewandelt wird, und wobei das Verfahren dadurch gekennzeichnet ist, daß der Schritt des Lichtsammelns aus dem Bereich die Maßnahme umfaßt, daß Licht aus dem Bereich zurück zu dem drehbaren Abtastelement, das den Abtastlaserstrahl bildet, über einen Reflektor reflektiert wird, der ein konjugiertes Bild der Pupille an der Facette bildet, welches Bild die Facette überfüllt, so daß die Facette nur einen Teil des gesammelten Lichts zum Detektor zur Entwicklung des zeitvariierenden Ausgangssignals zurückleitet.

39. Verfahren nach Anspruch 38, gekennzeichnet durch den weiteren Schritt des Ausgleichens von Abtastastigmatismus.

40. Verfahren nach Anspruch 38, gekennzeichnet durch die weiteren Schritte, daß das Objekt mit dem Eintrittsabtaststrahl durch eine gewählte kleine Eintrittsöffnung beleuchtet, der Detektor mit dem Austrittsstrahl durch eine gewählte große, mit der Eintrittsöffnung konzentrische Austrittsöffnung beleuchtet, die Facette des ersten Abtastgeräts an einer konjugierten Ebene der Austrittsöffnung angeordnet und der Detektor an einer konjugierten Ebene der Austrittsöffnung angeordnet wird.

41. Verfahren nach Anspruch 38, gekennzeichnet durch den weiteren Schritt von Erkennungsmitteln zum Feststellen des Austrittsstrahls an einer konjugierten Ebene des Augenfundus.

**Revendications**

1. Ophtalmoscope à balayage pour fournir une représentation de sortie à deux dimensions du caractéristiques de réflexion de la région postérieure de l'œil, cet ophtalmoscope comprenant une source de faisceau laser (11) pour produire un faisceau laser ayant une aire de section transversale définie qui est petite comparativement à la région (19a) qui doit être balayée, et à la pupille de l'œil, un système optique pour diriger ce faisceau laser à travers la pupille de l'œil, vers et sur la zone du fond de cet œil, ce système optique comportant un premier élément de balayage comprenant un élément rotatif (15) présentant des facettes espacées pour modifier la direction d'un faisceau laser incident et des moyens d'entraînement pour faire tourner cet élément de telle façon qu'une facette de celui-ci provoque le balayage du faisceau laser suivant une première coordonnée, un moyen réflecteur (18) pour diriger le faisceau laser, à partir de ladite facette du premier élément de balayage, à travers une portion de la pupille de l'œil, vers et sur la région (19a), en produisant ainsi un balayage du faisceau laser en travers de cette région, un moyen pour recueillir la lumière en provenance de cette région, à travers une ouverture de sortie dans la pupille de l'œil, et pour diriger cette lumière, par l'intermédiaire du moyen réflecteur (18), en direction d'un détecteur (21), afin de produire un signal de sortie variant dans le temps correspondant à la fréquence de balayage du faisceau laser incident, un moyen (14) de séparation du faisceau à travers lequel passent le faisceau laser et la lumière recueillie, et un moyen de sortie (23) pour recevoir le signal de sortie et fournir une représentation de sortie en deux dimensions de la région balayée, cet ophtalmoscope étant caractérisé en ce que le moyen réflecteur est disposé de manière à produire une image conjuguée de la pupille de l'œil à l'endroit de ladite facette, et la lumière collectée à partir de ladite région est dirigée en arrière vers et sur l'élément rotatif de telle façon que l'image conjuguée de la pupille déborde ladite facette si bien que cette facette redirige uniquement une portion de la lumière collectée en direction du détecteur (21), afin de produire le signal de sortie variant dans le temps.

2. Ophtalmoscope suivant la revendication 1, caractérisé en ce que le premier élément de balayage a une dimension dans le sens de la coordonnée du balayage et un trajet de déplacement de chaque facette de l'élément de balayage tels que chaque facette se déplace pratiquement en travers de la totalité de la section droite du faisceau réfléchi, dans le sens de la coordonnée du balayage, tout en réfléchissant la lumière en arrière en direction du détecteur sur lequel elle tombe.

3. Ophtalmoscope suivant l'une quelconque des revendications 1 ou 2, caractérisé en ce que le moyen réflecteur est un miroir de focalisation (18).

4. Ophtalmoscope suivant l'une quelconque des revendications précédentes, caractérisé en ce que le moyen de séparation du faisceau (14) est disposé de manière à diriger le faisceau laser d'entrée en direction du premier élément de balayage et à diriger la lumière réfléchie, provenant de la région balayée, vers le détecteur (21), le moyen séparateur de faisceau comportant un écran central pour la lumière réfléchie, cet écran central ayant sensiblement la même dimension de section transversale que celle de la section transversale du faisceau laser d'entrée, le moyen détecteur (21) étant disposé optiquement au-delà du moyen de séparation du faisceau de manière à recevoir la lumière réfléchie à partir de l'élément rotatif laquelle passe vers l'arrière le long du trajet en direction du faisceau, en dehors de la position centrale interceptée.

5. Ophtalmoscope suivant l'une quelconque des revendications précédentes, caractérisé en ce que le premier élément de balayage est un polygone à plusieurs facettes.

6. Ophtalmoscope suivant la revendication 3, caractérisé en ce que le miroir de focalisation est basculé autour d'un axe, la position relative de ce miroir de focalisation le long de l'axe optique étant ajustée de manière à compenser l'astigmatisme produit par le basculement dans la section droite pupillaire de l'enveloppe du faisceau effectuant le balayage.

7. Ophtalmoscope suivant la revendication 5, caractérisé en ce que la dimension de chaque facette du polygone, le long de la coordonnée de balayage, la dimension du faisceau réfléchi à partir du fond de l'œil, à la surface de la facette du polygone, et le trajet de déplacement de chaque facette du polygone le long de la coordonnée du balayage sont tels qu'une facette unique effectue un balayage en travers de la totalité de l'aire de la section transversale du faisceau, dans la direction du balayage, tout en réfléchissant la lumière vers l'arrière en direction du détecteur.

8. Ophtalmoscope suivant l'une quelconque des revendications précédentes, caractérisé en ce que le moyen de séparation du faisceau (14) est un miroir de déviation ayant une surface réfléchissante juste suffisamment grande pour englober l'aire de section transversale définie du faisceau laser, ce miroir de déviation étant disposé de manière à intercepter le faisceau laser et à le rediriger vers et sur le premier élément de balayage, ce miroir de déviation étant placé de manière à agir en tant qu'écran central pour la lumière réfléchie passant en direction du détecteur.

9. Ophtalmoscope suivant l'une quelconque des revendications précédentes, caractérisé en ce qu'un second élément de balayage (17) est disposé, en étant aligné optiquement, entre le premier élément de balayage et l'œil devant être balayé, afin de déplacer le faisceau laser de balayage dans une direction perpendiculaire à la première coordonnée, afin d'effectuer un balayage en deux dimensions de la zone du fond de l'œil.

10. Ophtalmoscope suivant la revendication 9 dépendant de la revendication 3, caractérisé en ce qu'il comprend un second miroir de focalisation (16) placé entre les éléments de balayage (15, 17) en tant que relais conjugué pour diriger le faisceau laser réfléchi à partir de la facette du premier élément de balayage (15) vers et sur la surface de ce second miroir de focalisation, l'un des miroirs de focalisation étant basculé autour d'un axe, les positions relatives de ces miroirs de focalisation le long de l'axe optique étant ajustées de manière à compenser l'astigmatisme produit par le basculement dans la section droite pupillaire de l'enveloppe du faisceau effectuant le balayage.

11. Ophtalmoscope suivant l'une quelconque des revendications précédentes, caractérisé en ce que le faisceau laser a une longueur d'onde comprise dans la gamme infrarouge.

12. Ophtalmoscope suivant l'une quelconque des revendications précédentes, caractérisé en ce qu'il comprend un écran formant diaphragme (26) placé à l'endroit du plan conjugué de la rétine, dans le faisceau réfléchi, au-delà d'un miroir de déviation, le moyen détecteur (21) étant situé optiquement au-delà de cet écran formant diaphragme à l'endroit d'un plan conjugué de l'image pupillaire, afin de recevoir la lumière réfléchie à partir de l'élément de balayage qui passe vers l'arrière le long du trajet en direction du miroir de déviation, ce miroir de déviation constituant un écran uniquement pour la portion centrale du faisceau réfléchi.

13. Ophtalmoscope suivant la revendication 12, caractérisé en ce que l'écran formant diaphragme est construit de manière à avoir une ouverture variable.

14. Ophtalmoscope suivant l'une quelconque des revendications précédentes, caractérisé en ce qu'il comprend un modulateur opto-acoustique (41) disposé de manière à moduler le faisceau laser et un moyen à programme (34) pour programmer la modulation du faisceau laser par le modulateur opto-acoustique, afin de présenter des tracés graphiques spécifiques lors du balayage de la zone du fond de l'œil.

15. Ophtalmoscope suivant la revendication 14, caractérisé en ce qu'il comporte des prismes (32) couplés optiquement avec le modulateur opto-acoustique (41) afin de donner pratiquement le même angle d'émission de faisceaux laser de différentes longueurs d'onde incidentes en provenance du modulateur opto-acoustique, tout en préservant en même temps la relation de Bragg pour les différentes longueurs d'onde dans le modulateur opto-acoustique.

16. Ophtalmoscope suivant la revendication 9, caractérisé en ce que le réflecteur polygonal (15) est entraîné en rotation à une vitesse permettant d'obtenir une fréquence de balayage, le long de la première coordonnée, sur la zone du fond de l'œil, de 15,75 kHz et le second élément de balayage (17) produit un mouvement de balayage dans une direction perpendiculaire à la première coordonnée, à une fréquence d'environ 50,4 ou 60 Hertz, et les moyens d'affichage comportent un dispositif à trame de télévision.

17. Ophtalmoscope suivant la revendication 1, caractérisé en ce que le faisceau laser de balayage est dirigé à travers un point pivot dans un plan ayant un emplacement sélectionné par rapport au faisceau laser pour introduire le faisceau laser de balayage vers et dans l'œil en cours d'examen, à travers seulement une petite portion de la pupille de l'œil, et le faisceau de balayage se déplace à partir de ce point pivot vers et sur une région de grand angle du fond de l'œil disposé avec la pupille de l'œil à l'endroit dudit plan sélectionné.

18. Ophtalmoscope suivant la revendication 15, caractérisé en ce que la dimension de l'aire de la section transversale du faisceau réfléchi est définie par l'image à l'endroit d'un plan conjugué de l'ouverture de sortie du système, l'ouverture de sortie de ce système étant notablement plus grande que l'ouverture d'entrée pour le faisceau laser de balayage.

19. Ophtalmoscope suivant l'une quelconque des revendications précédentes, caractérisé en ce que le détecteur est placé de manière à recevoir pratiquement uniquement la lumière réfléchie directement à partir de la portion spécifique de la zone balayée qui est éclairée par le faisceau laser à un instant donné quelconque.

20. Ophtalmoscope suivant l'une quelconque des revendications 1 à 18, caractérisé en ce que le détecteur est placé de manière à recevoir uniquement la lumière réfléchie à partir d'une portion spécifique de la zone balayée qui est éclairée indirectement à partir de la portion balayée qui est éclairée directement à un instant donné quelconque.

21. Ophtalmoscope suivant la revendication 1, caractérisé en ce que l'ouverture de sortie est définie par la dimension de la pupille de l'œil.

22. Ophtalmoscope suivant l'une quelconque des revendications précédentes, caractérisé en ce qu'il comprend des moyens pour détecter la position des facettes successives du premier élément de balayage et pour fournir un signal de position indiquant l'instant où chaque facette successive occupe la même position, ce signal de position étant fourni au moyen d'affichage afin de commander la synchronisation des lignes horizontales produites sur ce moyen d'affichage.

23. Ophtalmoscope suivant la revendication 22, caractérisé en ce que le moyen d'affichage est du type à trame de télévision à deux dimensions.

24. Ophtalmoscope suivant l'une quelconque des revendications précédentes, caractérisé en ce qu'il comprend des moyens pour arrêter le balayage du fond de l'œil par le faisceau laser si un défaut de sécurité est détecté dans l'ophtalmoscope.

25. Ophtalmoscope suivant la revendication 22, caractérisé en ce qu'il comprend des moyens pour arrêter le balayage du fond de l'œil par le faisceau laser et le signal de position est fourni à ces moyens pour arrêter le faisceau laser, afin d'empêcher le balayage par le faisceau laser pendant la période de temps durant laquelle la trace horizontale n'est pas affichée.

26. Ophtalmoscope suivant l'une quelconque des revendications précédentes, caractérisé en ce que le moyen détecteur est une diode à avalanche.

27. Ophtalmoscope suivant la revendication 1, caractérisé en ce qu'il comporte des moyens à plate-forme pour supporter l'ophtalmoscope et permettre le mouvement de cet ophtalmoscope afin de modifier l'angle suivant lequel le faisceau laser pénètre à travers la pupille de l'œil, sans changer les positions relatives de la source de faisceau laser, du système optique et du détecteur. de faisceau, ces moyens à plate-forme étant montés de manière à permettre un mouvement de translation de l'ophtalmoscope uniquement suivant deux axes perpendiculaires à l'axe de rotation de l'élément rotatif et suivant un axe parallèle à cet axe de rotation, et pour permettre un mouvement de rotation de l'ophtalmoscope uniquement autour d'un axe parallèle à cet axe de rotation.

28. Ophtalmoscope suivant la revendication 14, caractérisé en ce qu'il comprend un circuit de commande, le modulateur opto-acoustique comporte un circuit d'attaque électrique et un transducteur électromécanique, ce circuit d'attaque commandant les caractéristiques optiques du transducteur de manière à permettre au faisceau laser de tomber sur le premier élément de balayage ou bien d'être dévié à l'écart de ce premier élément de balayage, et le circuit d'attaque comprend un oscillateur haute fréquence, un modulateur équilibré ayant une entrée vidéo, et un circuit de couplage assurant le couplage entre l'oscillateur haute fréquence et le modulateur, ce circuit de commande fournissant des signaux à ladite entrée vidéo conformément à un programme en provenance des moyens à programme.

29. Ophtalmoscope suivant la revendication 21, caractérisé en ce que le modulateur opto-acoustique comporte un circuit d'attaque électrique et un transducteur électromécanique, ce circuit d'attaque commandant les caractéristiques optiques du transducteur de manière à permettre au faisceau laser de tomber sur le premier élément de balayage ou bien d'être dévié à l'écart de ce premier élément de balayage, et le circuit d'attaque comprend un oscillateur haute fréquence, un modulateur équilibré ayant une entrée vidéo, et un circuit de couplage assurant le couplage entre l'oscillateur haute fréquence et le modulateur, ce faisceau laser étant empêché d'effectuer le balayage en interrompant le couplage entre l'oscillateur haute fréquence et le modulateur équilibré.

30. Ophtalmoscope suivant l'une quelconque des revendications 1 à 10, caractérisé en ce qu'il comprend un télescope, ayant un grossissement différent de un, disposé entre le moyen réflecteur et la pupille de l'œil, pour ajuster le foyer du faisceau sur la zone du fond de l'œil.

31. Ophtalmoscope suivant l'une quelconque des revendications précédentes, caractérisé en ce que le moyen de sortie comporte un moyen d'affichage pour afficher une image en réponse au signal variant dans le temps et pour obtenir des variations de ladite image correspondant à des variations de la lumière directement réfléchie à partir de l'objet balayé.

32. Ophtalmoscope suivant l'une quelconque des revendications précédentes, caractérisé en ce qu'un second élément de balayage est placé sur le trajet optique du faisceau d'entrée à partir de la source et du premier élément de balayage, afin de diriger le faisceau d'entrée vers et sur l'objet devant être balayé et pour déplacer ce faisceau d'entrée dans une direction perpendiculaire à la direction de la première coordonnée.

33. Ophtalmoscope suivant la revendication 32, caractérisé en ce que le second élément de balayage comporte un galvanomètre réfléchissant.

34. Ophtalmoscope suivant la revendication 5, caractérisé en ce que la vitesse de rotation du réflecteur polygonal est telle que la fréquence de balayage du faisceau d'entrée est sensiblement de 15,75 kHz et le moyen de sortie comporte un générateur d'image à trame du type télévision.

35. Ophtalmoscope suivant la revendication 34, caractérisé en ce qu'il comporte un second élément de balayage placé sur le trajet optique du faisceau d'entrée à partir de la source et du premier élément de balayage, afin de diriger le faisceau d'entrée vers et sur l'objet devant être balayé et de déplacer le faisceau d'entrée dans une direction perpendiculaire à la direction de la première coordonnée, ce second élément de balayage fonctionnant de manière à produire une fréquence de balayage, dans la direction perpendiculaire à la direction de la première coordonnée, qui est sensiblement de 50,4 ou 60 Hz correspondant à une fréquence de trame de télévision.

36. Ophtalmoscope suivant la revendication 35, caractérisé en ce que le réflecteur polygonal rotatif comporte un nombre de facettes qui est divisible régulièrement en 525.

37. Ophtalmoscope suivant la revendication 31, caractérisé en ce que le moyen de sortie comporte un générateur d'image à trame à lignes multiples et le réflecteur polygonal rotatif comporte un nombre de facettes qui est divisible régulièrement pour donner le nombre des lignes de la trame affichée.

38. Procédé pour former une représentation de sortie à deux dimensions des caractéristiques de réflexion d'une région postérieure de l'œil, ce procédé comprenant les étapes consistant à produire un faisceau laser ayant une aire de section transversale définie qui est faible comparativement à la région devant être balayée et à la pupille de l'œil, à diriger ce faisceau laser à travers la pupille de l'œil, vers et sur ladite région, en dirigeant ce faisceau sur une facette d'un élément de balayage rotatif, afin de former un faisceau laser de balayage le long d'une première coordonnée, et à réfléchir le faisceau laser de balayage, à travers une portion de la pupille de l'œil, vers et sur ladite région postérieure, à collecter la lumière en provenance de cette région, à travers la pupille, et à la réfléchir en arrière vers un photodétecteur, afin de produire un signal de sortie variant dans le temps corrélé avec la fréquence de balayage du faisceau laser, et à convertir le signal de sortie en une représentation à deux dimensions de la région balayée, ce procédé étant caractérisé en ce que l'étape de collecte de la lumière à partir de ladite région consiste à réfléchir la lumière à partir de la région, en arrière, en direction de l'élément de balayage rotatif qui forme le faisceau laser de balayage, par l'intermédiaire d'un réflecteur qui forme une image conjuguée de la pupille à l'endroit de ladite facette, cette image débordant ladite facette si bien que la facette retransmet uniquement une portion de la lumière collectée en direction du détecteur afin de produire le signal de sortie variant dans le temps.

39. Procédé suivant la revendication 38, caractérisé en ce qu'il comprend une étape additionnelle de compensation de l'astigmatisme du balayage.

40. Procédé suivant la revendication 38, caractérisé en ce qu'il comprend les étapes additionnelles consistant à éclairer l'objet avec le faisceau d'entrée de balayage à travers une petite ouverture d'entrée sélectionnée, à éclairer le détecteur avec le faisceau de sortie à travers une grande ouverture de sortie sélectionnée, concentrique avec l'ouverture d'entrée, à placer la facette du premier dispositif de balayage dans un plan conjugué de l'ouverture de sortie, et à placer le détecteur dans un plan conjugué de l'ouverture de sortie.

41. Procédé suivant la revendication 38, caractérisé en ce qu'il comprend l'étape additionnelle consistant à placer des moyens de détection du faisceau de sortie à l'endroit d'un plan conjugué du fond de l'œil.

*FIG. 1*

*FIG. 2*

*FIG. 3*

*FIG. 4*

*FIG. 5*

*FIG. 6*

*FIG. 6A*

## FIG. 7

## FIG. 8

## FIG. 9

**FIG. 10**

**FIG. 10A**

**FIG. 11**

**FIG. 12**

*FIG. 13*

EP 0 223 356 B1